# EUROPEAN PATENT APPLICATION

(11) **EP 3 369 369 A1**
(43) Date of publication of application: **05.09.2018**
(21) Application number: 18159001.9
(22) Date of filing: 27.02.2018
(51) Int. Cl.: A61B 5/00, A61B 5/053

(54) **PATHOGEN DETECTION**

(30) Priority: 28.02.2017 US 201762464402 P; 09.02.2018 US 201815892765
(71) Applicant: Covidien LP, Mansfield, MA 02048 (US)
(72) Inventor: SHALTIS, Phillip, Sharon, MA 02067 (US)
(74) Representative: Prock, Thomas

(57) **Abstract**

In some examples, a pathogen detection sensor includes an electrical circuit changeable from a first impedance state to a second impedance state in response to a change in the presence of pathogens (e.g., in response to pathogen growth). A substrate of a vascular access includes at least a part of the pathogen detection sensor, such as, for example, part of the electrical circuit. In this way, the pathogen detection sensor may be positionable proximate to a vascular access site in order to detect the presence of pathogens at or near a vascular access site of a patient.

## Description

### CROSS-REFERENCE TO RELATED APPLICATION

This application claims the benefit of and priority to U.S. Provisional Patent Application No. 62/464,402 filed February 28, 2017, the entire disclosure of which is incorporated by reference herein.

### BACKGROUND

A vascular access system may include an access line, such as a catheter, that is inserted through skin of the patient at a vascular access site and into the patient's vasculature. Growth of pathogens at or near the vascular access site over time may lead to an infection at the vascular access site or a bloodstream infection.

### SUMMARY

This disclosure describes devices, systems, and techniques for detecting the presence of pathogens at or near, for example, a vascular access site of a patient. As described herein, a pathogen detection sensor includes an electrical circuit changeable from a first impedance state to a second impedance state in response to a change in the presence of pathogens (e.g., in response to pathogen growth). A substrate of a vascular access system includes at least a part of the pathogen detection sensor, such as, for example, part of the electrical circuit. In this way, the pathogen detection sensor may be positionable proximate to a vascular access site in order to detect the presence of pathogens at or near a vascular access site of a patient.

Clause 1: In some examples, a pathogen detection system comprises an electrical circuit changeable, from a first impedance state to a second impedance state, in response to a pathogen, the electrical circuit comprising: a first electrical contact; and a second electrical contact electrically connectable to the first electrical contact by the presence of a pathogen extending between the first electrical contact and the second electrical contact, wherein the electrical circuit is in the second impedance state when the first and second electrical contacts are electrically connected to each other through the presence of the pathogen.

Clause 2: In some examples of the pathogen detection system of clause 1, the electrical circuit comprises a flexible circuit.

Clause 3: In some examples of the pathogen detection system of clause 1 or clause 2, the first electrical contact and the second electrical contact each comprise respective electrically conductive traces.

Clause 4: In some examples of the pathogen detection system of any of clauses 1-3, the first electrical contact and the second electrical contact each comprise a biocompatible metal.

Clause 5: In some examples of the pathogen detection system of clause 4, the biocompatible metal comprises at least one of silver and gold.

Clause 6: In some examples of the pathogen detection system of any of clauses 1-5, the electrical circuit further comprises electrically conductive particles within an electrically nonconductive substrate, the electrically conductive particles disposed between the first electrical contact and the second electrical contact.

Clause 7: In some examples of the pathogen detection system of clause 6, the conductive particles comprise a biocompatible metal.

Clause 8: In some examples of the pathogen detection system of clause 7, the biocompatible metal comprises at least one of silver and gold.

Clause 9: In some examples of the pathogen detection system of any of clauses 1-8, the system further comprises a substrate having a first side and a second side opposite the first side, the substrate disposed between the first electrical contact and the second electrical contact.

Clause 10: In some examples of the pathogen detection system of any of clauses 1-9, the substrate defines an opening extending from the first side of the substrate to the second side of the substrate, the first electrical contact comprising a first layer of conductive material on the first side of the substrate, and the second electrical contact comprising a second layer of conductive material on the second side of the substrate, wherein the first and second electrical contacts are electrically connectable to one another through growth of the pathogen through the opening.

Clause 11: In some examples of the pathogen detection system of any of clauses 1-9, the first and second electrical contacts are on a same one of the first side or the second side of the substrate.

Clause 12: In some examples of the pathogen detection system of any of clauses 1-9, the substrate comprises a protective cover.

Clause 13: In some examples of the pathogen detection system of any of clauses 1-9, the substrate comprises a hemostatic patch.

Clause 14: In some examples of the pathogen detection system of any of clauses 1-13, the system further comprises an access line coupled to the substrate, wherein the access line is introducible into vascular of the patient.

Clause 15: In some examples of the pathogen detection system of clause 14, the substrate comprises an antimicrobial collar disposed about a circumference of the access line, the first electrical contact and the second electrical contact each positioned along the antimicrobial collar.

Clause 16: In some examples of the pathogen detection system of any of clauses 1-15, the system further comprises an electrically insulative layer over the first electrical contact and over the second electrical contact, the electrically insulative layer permeable to growth of the pathogen.

Clause 17: In some examples of the pathogen detection system of any of clauses 1-16, the electrical circuit further comprises a radio frequency antenna in electrical communication with the first electrical contact and the second electrical contact.

Clause 18: In some examples of the pathogen detection system of clause 17, the system further comprises a reader device configured to transmit an interrogation signal that energizes the radio frequency antenna, wherein, in response to the interrogation signal, the radio frequency antenna is configured to transmit a status signal to the reader device, and the reader device is configured to determine, based on the received status signal, whether the electrical circuit is in the first impedance state or in the second impedance state.

Clause 19: In some examples of the pathogen detection system of clause 17, the radiofrequency antenna is a passive radiofrequency antenna.

Clause 20: In some examples of the pathogen detection system of any of clauses 1-19, the system further comprises a light emitter and a power source, wherein electrical communication between the light emitter and the power source is dependent upon whether the electrical circuit is in the first impedance state or in a second impedance state.

Clause 21: In some examples of the pathogen detection system of any of clauses 1-20, the system further comprises a sound emitter and a power source, wherein electrical communication between the sound emitter and the power source is dependent upon whether the electrical circuit is in the first impedance state or in the second impedance state.

Clause 22: In some examples of the pathogen detection system of any of clauses 1-21, the system further comprises means for communicating the second impedance state of the electrical circuit.

Clause 23: In some examples of the pathogen detection system of any of clauses 1-21, the system further comprises a system configured to communicate the second impedance state of the electrical circuit to a remote device.

Clause 24: In some examples of the pathogen detection system of any of clauses 1-23, the electrical circuit is open in the first impedance state and closed in the second impedance state.

Clause 25: A method of making any of the systems of clauses 1-24.

Clause 26: A method of using any of the systems of clauses 1-24.

Clause 27: In some examples, a method comprises receiving an interrogation signal at a radio frequency antenna of an electrical circuit changeable from a first impedance state to a second impedance state in response to presence of a pathogen; and transmitting, in response to the interrogation signal, a response signal from the radio frequency antenna, the response signal indicative of whether the electrical circuit is in the first impedance state or in the second impedance state.

Clause 28: In some examples of the method of clause 26, the interrogation signal powers the radio frequency antenna and electrical circuit.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

FIG. 1 illustrates an example vascular access system and an example vascular access site of a patient.
FIG. 2 is a conceptual diagram of an example electrical circuit of a pathogen detection sensor of a substrate of a vascular access system.
FIG. 3 illustrates a perspective view of an example hemostatic patch including at least a part of the electrical circuit of FIG. 2.
FIG. 4 illustrates a perspective view of an access line and the hemostatic patch of FIG. 3.
FIG. 5 illustrates an example protective cover of a vascular access system, where the protective cover includes a radio frequency antenna.
FIG. 6 is a schematic diagram showing the electrical circuit of FIG. 2 in an example first impedance state.
FIG. 7 is a schematic diagram showing the electrical circuit of FIG. 2 in an example second impedance state.
FIG. 8 is a side elevation view of an example hemostatic patch that includes electrically conductive traces that may be part of the electrical circuit of FIG. 2.
FIGS. 9A and 9B illustrate another example configuration of electrically conductive traces that may define part of the electrical circuit of FIG. 2.
FIG. 10 illustrates an example electrically insulative layer that may be positioned over the electrical contacts of the electrical circuit of FIG. 2.
FIG. 11 is a side elevation view of an example hemostatic patch that includes a first electrical contact, a second electrical contact, and electrically conductive particles in an electrically nonconductive substrate.
FIG. 12 is a schematic diagram showing an electrical circuit including the first and second electrical contacts of FIG. 11, where the electrical circuit is in a first impedance state.
FIG. 13 illustrates an example bacterial biofilm on the hemostatic patch of FIG. 11.
FIG. 14 is a schematic diagram showing the electrical circuit including the first and second electrical contacts of FIG. 11, where the electrical circuit is in a second impedance state.
FIGS. 15A and 15B illustrate a side elevation view of an example access line including electrically conductive traces that may define a part of the electrical circuit of FIG. 2.
FIG. 16 is a circuit diagram conceptually illustrating a first impedance state of the electrical circuit including the electrically conductive traces of FIGS. 15A and 15B when the electrically conductive traces are not electrically connected.
FIG. 17 is a conceptual illustration of an example bacterial biofilm on the access line of FIGS. 15A and 15B.
FIG. 18 is a circuit diagram conceptually illustrating a second impedance state of the electrical circuit including the electrically conductive traces of FIGS. 15A and 15B when the electrically conductive traces are electrically connected.

### DETAILED DESCRIPTION

In examples described here, a substrate of a vascular access system, such as a hemostatic patch, a protective cover, or an access line, includes a pathogen detection sensor that can detect the presence of pathogens at or near a vascular access site of a patient. The pathogen detection sensor includes an electrical circuit changeable (e.g., switchable) from a first impedance state to a second impedance state, different from the first impedance state, in response to a change in the presence of pathogens (e.g., in response to pathogen growth). Thus, the second impedance state of the electrical circuit may indicate the potential presence of pathogens on a substrate of the vascular access system, whereas the first impedance state of the electrical circuit may indicate pathogens are not detected by the pathogen detection sensor.

While a pathogen growth in the form of a bacterial biofilm is primarily referred to herein the pathogen growth may additionally or alternatively take another form. The pathogen detection sensors described herein can, in some examples, detect the presence of pathogens in forms other than a bacterial biofilm.

The pathogens that may be detected using the devices, systems, and techniques described herein may be in the form of bacterial colonizations at or near a vascular access site, where the bacterial cells are electrically conductive. Different bacteria may exhibit different degrees of electrical conductivity. *Staphylococcus aureus* is an example of a pathogen that has been found to decrease the impedance of a control sample when allowed to grow, e.g., over a two day period. In some cases, the impedance of the sample including *Staphylococcus aureus* may be in a range of about 80 ohms (Ω) to about 250 Ω □□□ However, the specific measured impedances of a bacterial colonization may be a function of one or more factors, including, but not limited to, the configuration of the sensor used to measure impedance, the bacterial type of interest, the method selected for impedance monitoring (e.g., direct-current (DC), alternating current (AC), frequency of input, and the like). The dynamic range and measurements expected from a pathogen sensor may be dependent on the nature of the voltage/current input. For examples, if a DC signal used, the sensor may detect presence of a pathogen and/or a pathogen growth by at least detecting a voltage/current drop resulting from the resistance of the pathogen. However, if an AC signal is used, then the sensor may detect presence of a pathogen and/or a pathogen growth by at least detecting a phase change between input and output signals.

Some types of bacteria may go through different phases of growth, including, for example, a lag phase, log phase, stationary phase, and death phase. During some of these growth phases, such as the lag phase and the log phase, the impedance of the bacterial growth may decrease as the number of cells in the bacterial colonization increases. The impedance of the bacterial growth may eventually reach a substantially steady state impedance, e.g., during a stationary phase of the bacterial growth. The pathogen detection sensors described herein may detect a pathogen grown in any of the lag phase, the log phase, the stationary phase, or the death phase, provided the quantity of bacteria is sufficient to cause the change in impedance state of the electrical circuit of the respective sensor.

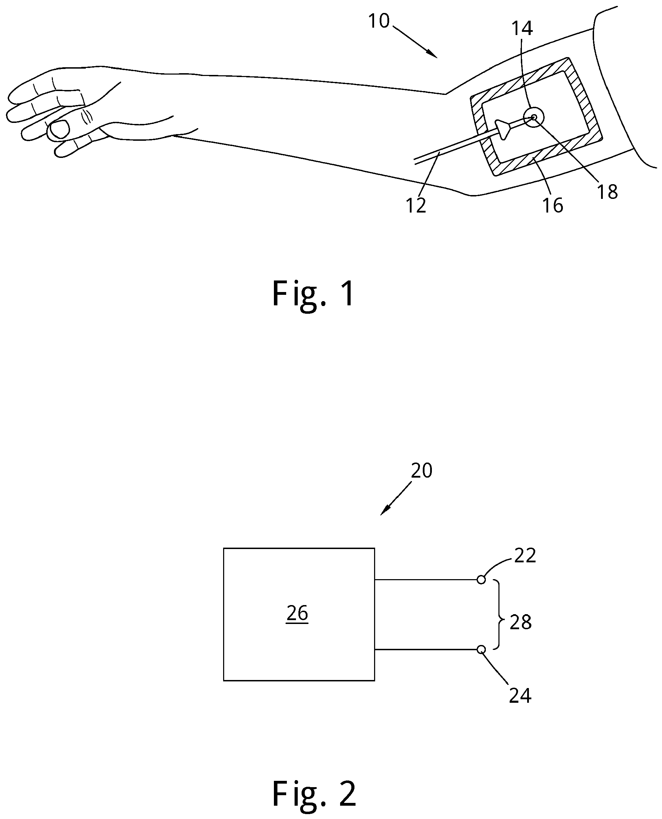

As discussed above, in some examples described herein, a substrate of a vascular access system comprises a pathogen detection sensor that can detect the presence of pathogens at or near a vascular access site of a patient based on the impedance of the pathogens. FIG. 1 illustrates an example vascular access system 10 and an example vascular access site 18 of a patient. The vascular access system 10 includes an access line 12, a hemostatic patch 14, and a protective cover 16. The access line 12 can be, for example, a medical catheter, that delivers saline, medication, or other therapeutic agents to the patient. The access line 12 is introduced within vasculature of the patient at the vascular access site 18 in an arm of the patient. Although FIG. 1 illustrates the vascular access site 18 in an arm of a patient, in other examples, the vascular access site 18 may be otherwise located, e.g., in a leg or chest of the patient.

After the access line 12 is introduced into vasculature of the patient via the vascular access site 18, and the patient's skin around the access site 18 has been cleaned, the hemostatic patch 14, the protective cover 16, or both the hemostatic patch 14 and the protective cover 16, may be placed over the top of the vascular access site 18 to physically secure the access line 12 relative to the patient, and to protect the vascular access site 18 from external pathogens. If both the hemostatic patch 14 and the protective cover 16 are used, then the hemostatic patch 14 may be positioned between the patient's skin and the protective cover 16.

In some cases, pathogens may remain at the vascular access site 18, despite attempts to disinfect the area. If not detected early, the pathogens present at the vascular access site 18 may replicate and lead to an infection at the site. A substrate of the vascular access system 10 includes a pathogen detection sensor that can detect the presence of pathogens at the vascular access site 18 and notify a care provider. The notification may allow the care provider to take a responsive action prior to a need for more significant interventions and prior to, for example, development of an infection due to the pathogens at the vascular access site 18.

The pathogen detection sensor includes an electrical circuit changeable from a first impedance state to a second impedance state in response to a pathogen growth. FIG. 2 is a conceptual diagram of an example electrical circuit 20 of a pathogen detection sensor of vascular access system 10. The electrical circuit 20 comprises a first electrical contact 22, a second electrical contact 24, and a communication module 26.

The second electrical contact 24 is electrically connectable to the first electrical contact 22 by a pathogen in a region 28 extending between the first and second electrical contacts 22, 24 and electrically connecting the contacts 22, 24. As discussed below, the region 28 can be a surface of a substrate of the vascular access system 10 (FIG. 1), such as the hemostatic patch 14, the protective cover 16, the access line 12, or another substrate.

The electrical circuit 20 may be in a first impedance state (e.g., an open state or a relatively high impedance state) when the first and second electrical contacts 22, 24 are not in electrical communication (e.g., electrically connected) with each other, and in a second impedance state (e.g., a closed state or a relatively low impedance state) when the first and second electrical contacts 22, 24 are electrically connected to each other, e.g., by the pathogen growth. The electrical circuit 20 has a lower impedance when in the second impedance state compared to when the electrical circuit 20 is in the first impedance state. The second impedance state of the electrical circuit 20 indicates the potential presence of pathogens in the region 28 of a substrate, whereas the first impedance state of the electrical circuit 20 may indicate pathogens are not detected in the region 28, e.g., are not present in a quantity sufficient to change the electrical circuit 20 to the second impedance state.

In some examples, the second electrical contact 24 is electrically isolated from the first electrical contact 22 in the absence of the pathogen growth or another electrically conductive pathway separate from the electrical circuit 20 itself and electrically connecting the first and second electrical contacts 22, 24. For example, the surface of the vascular access system substrate between the electrical contacts 22, 24 can be formed from an electrically insulative material that electrically isolates the electrical contacts 22, 24 in the absence of a pathogen or other electrically conductive material separate from the substrate within the region 28 between the contacts 22, 24 and electrically connecting the contacts 22, 24.

In some examples, the electrical circuit 20 can be a relatively low-profile, flexible circuit, such as a thin-film flexible circuit. This may help minimize the obtrusiveness of the pathogen detection sensor of the substrate of the vascular access system. In addition, compared to a pathogen detection sensor that is physically separate from an existing substrate of the vascular access system 10, the pathogen detection sensor including the electrical circuit 20 that is attached to substrate of the vascular access system 10 may improve the ease with which the pathogen detection system may be used. The pathogen detection sensors described herein may eliminate the need for a care provider to attach a separate pathogen detection sensor to a patient, which may add a step to the vascular access procedure. In addition, because the pathogen detection sensors described herein are attached to a vascular access system substrate, the pathogen detection sensors can be properly placed relative to the vascular access site 18 by proper usage of the vascular access system 10. In this way, little to no additional training may be required to use the pathogen detection sensors described herein.

The impedance state of the electrical circuit 20 may be detected and communicated to a care provider using any suitable technique. In the example shown in FIG. 2, the communication module 26 comprises circuitry and, in some examples, one or more other components (e.g., a light emitter or a sound emitter) that can communicate the impedance state of the electrical circuit 20 to a care provider or another user.

In some examples, the communication module 26 includes a passive radio frequency antenna, such as a radio frequency identification (RFID) antenna. When an external reader device is within a predetermined range of the radio frequency antenna, and the radio frequency reader may transmit an interrogation signal (e.g., electromagnetic waves) that energizes the radio frequency antenna and, if the electrical circuit 20 is in the second impedance state, then the antenna may transmit a return signal to the reader device in response to receiving the interrogation signal. However, if the electrical circuit 20 is in the first impedance state, then the antenna may not transmit a return signal to the reader device. Thus, if the reader device does not receive a return signal from the electrical circuit 20 in response to an interrogation signal, then a processor of the reader device (or another device), a care provider, or both, may determine that pathogens are not present in a quantity sufficient to change electrical circuit from the first impedance state to the second impedance state. On the other hand, if the reader device receives a return signal from the electrical circuit 20 in response to an interrogation signal, then a processor of the reader device (or another device), a care provider, or both, may determine that pathogens have been detected by the pathogen detection sensor.

In another example, the external reader device can transmit an interrogation signal that energizes the radio frequency antenna and, if the electrical circuit 20 is in the second impedance state, then the resonant frequency of the response signal provided by the electrical circuit 20 matches a predetermined resonant frequency (e.g., a resonant frequency of the interrogation signal or a resonant frequency stored by the reader device). However, if the electrical circuit 20 is in the second impedance state, then the resonant frequency of the response signal provided by the electrical circuit 20 does not match the predetermined resonant frequency. Thus, in this example, if a processor of the reader device (or another device) determines that a resonant frequency of the response signal provided by the electrical circuit 20 matches the predetermined resonant frequency, then the processor may determine that the electrical circuit 20 is in the second impedance state and, therefore, pathogens have been detected by the pathogen detection sensor. On the other hand, if processor of the reader device (or another device) determines that a resonant frequency of the response signal provided by the electrical circuit 20 does not match the predetermined resonant frequency, then the processor may determine that pathogens are not present in a quantity sufficient to change electrical circuit from the first impedance state to the second impedance state.

In some examples, the reader device may transmit an interrogation signal that changes resonant frequency over time, such that the reader device sweeps a range of resonant frequencies, and determines that one or more resonant frequencies at which the reader device receives the desired response from the radio frequency antenna of the communication module 26. The one or more resonant frequencies at which the reader device receives the desired response may indicate the impedance state of the electrical circuit 20, and, therefore, may indicate whether the electrical circuit 20 is in a first impedance state (no pathogen detection) or in the second impedance state (pathogen detection). In some examples, the radio frequency antenna may provide a first response signal when the electrical circuit 20 is in the first impedance state, and a second response signal when the electrical circuit 20 is in the second impedance state. Thus, a user may verify that the electrical circuit 20 is working as intended in response to receiving a response signal, whether the received response is the first response signal or the second response signal.

A passively activated radio frequency antenna does not require a local power source (e.g., within the communication module 26) to operate, which may reduce the complexity of the vascular access system 10. In addition, in examples in which the communication module 26 is positioned on a substrate of the vascular access system 10, the absence of a local power source for energizing the radio frequency antenna may allow the substrate of the vascular access system to remain relatively low profile, which may be desirable for some patient settings.

In some examples, the reader device may be located proximate the patient and communicatively coupled to a remote device via a wired or wireless connection. In this way, the reader device can directly or indirectly, e.g., via a relay module, transmit an indication that the electrical circuit 20 is in the second impedance state to the remote device. The remote device can, in some examples, receive input from a plurality of pathogen detection sensors, e.g., associated with respective patients. In this way, a single care provider or a limited number of care providers may efficiently and remotely monitor vascular access systems of a plurality of patients for the presence of pathogens. In some examples, the remote device may be located at a central location (e.g., a nurse's station) and the patients and their respective vascular access systems may be located in their respective rooms.

In addition to, or instead of, the detection of the second impedance state of the circuit 20 indicating the presence of pathogens at or near the vascular access site 18 using a passive radio frequency antenna and reader device, the presence of pathogens can be communicated to a care provider using locally powered circuits. For example, the communication module 26 may include a visual or audible alerting mechanism that notifies a care provider of the change of the electrical circuit 20 from the first impedance state to the second impedance state, thereby notifying the care provider of the detection of pathogens by the sensor. For example, the communication module 26 may include a light emitter (e.g., a light emitting diode (LED), such as a micro-LED) or other user-perceivable indicator and a power source, and when the electrical circuit 20 is in the second impedance state, the light emitter electrically connected to the power source and the light emitter emits light. However, when the electrical circuit 20 is in the first impedance state, the light emitter is not electrically connected to the power source, and, therefore, does not emit light. Thus, a care provider may view the light emitter to quickly ascertain whether the sensor has detected pathogens.

The care provider notification provided by the pathogen detection sensor or another device (e.g., a RFID reader device) may allow a care provider to take a responsive action prior to a need for more significant interventions and prior to, for example, development of an infection, such as a central line-associated bloodstream infection (CLABSI) or a localized infection at the vascular access site. The responsive action can include, for example, cleaning the vascular access site 18, replacing one or more components of the vascular access system 10, or any combination thereof. In some examples, the detection of the presence of pathogens at or near the vascular access site 18 of a patient may be used to control the timing of the cleaning or other maintenance of the system 10, the vascular access site 18, or both. For example, a care provider may clean the vascular access site 18 in response to receiving notification from a pathogen detection sensor indicating the presence of pathogens at or near the vascular access site 18. In this way, the pathogen detection sensor may eliminate the need for unnecessary cleanings.

As discussed above, any suitable substrate of the vascular access system 10 that may be positioned proximate to the vascular access site 18 may include the pathogen detection sensor, and, therefore, at least a portion of the electrical circuit 20. In some examples, the hemostatic patch 14 of the vascular access system 10 comprises the pathogen detection sensor.

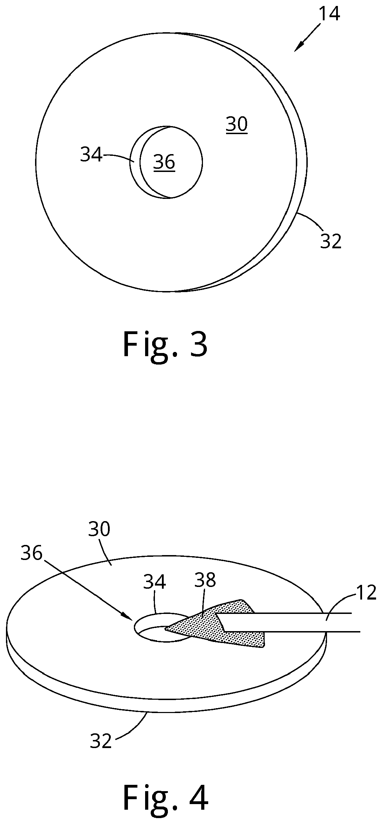

FIG. 3 illustrates a perspective view of an example hemostatic patch 14 including a pathogen detection sensor, and FIG. 4 illustrates a perspective view of the hemostatic patch 14 and the access line 12. The hemostatic patch 14 defines a first surface 30, a second surface 32 on an opposite side of the patch 14 from the first surface 30, a wall 34, and an opening 36. The wall 34 extends between the first surface 30 and the second surface 32, and defines a part of the opening 36. In some examples, the wall 34 may be substantially perpendicular to the first surface 30 and the second surface 32, though the wall 34 and the surfaces 30, 32 may have other relative orientations in other examples.

The second surface 32 of the hemostatic patch 14 may be a skin-facing surface of the patch 14 that includes one of one or more procoagulant materials, which may help control bleeding at the vascular access site 18. The hemostatic patch 14 may be positioned on the patient such that the second surface 32 is closer to the patient than the first surface 30, and such that the access line 12 extends through the opening 36, as shown in FIG. 4. In some examples, the hemostatic patch 14 can be secured in place with an adhesive, medical tape, or the like.

In the example shown in FIGS. 3 and 4, the hemostatic patch 14 includes a pathogen detection sensor, which includes at least a portion of the electrical circuit 20 (FIG. 2). In some examples, the first electrical contact 22 is located on the first surface 30 of the hemostatic patch 14, and the second electrical contact 24 is located on the second surface 32 of the hemostatic patch 14. For example, the first electrical contact 22 can be defined by continuous coating of an electrically conductive material on the first surface 30, and the second electrical contact 24 can be defined by continuous coating of an electrically conductive material on the second surface 32. The coatings may cover substantially the respective surface 30, 32, or at least a portion of the surfaces 30, 32 that is directly adjacent to the wall 34.

The electrical conductive material can be, for example, silver, gold, or another suitable biocompatible metal. Silver is relatively highly electrically conductive and biocompatible, and has natural anti-microbial properties. Thus, a silver coating on the first surface 30 and the second surface 32 of the hemostatic patch 14 may define a part of a pathogen detection sensor, while advantageously increasing the antibacterial resistance of the hemostatic patch 14.

As bacteria grows proximate the vascular access site 18, as shown conceptually in FIG. 4 as a bacterial biofilm 38, bacteria may transfer from the patient's skin surface to the top surface 22 of the hemostatic patch 14 through the opening 36 and along the wall 34. When the bacterial biofilm 38 contacts both the electrical contacts 20, 22 of the electrical circuit 20 on the first surface 30 and the second surface 32 of the hemostatic patch 14, respectively, the bacterial biofilm 38 may electrically connect the electrical contacts 20, 22, thereby changing (e.g., transitioning) the electrical circuit 20 from a first impedance state (e.g., an open state) to a second impedance state (e.g., a closed state). Detection of the second impedance state may indicate the presence of pathogens at or near the vascular access site 18.

In examples in which the communication module 26 of the circuit 20 includes a radio frequency antenna, the antenna may be placed around an outer periphery of the hemostatic patch 14, and may be in electrical communication with the contacts 22, 24. The radio frequency antenna may be, for example, positioned in an outer coating around the outer periphery of the hemostatic patch 14. In some examples, the radio frequency antenna may be formed by electrically conductive traces, where the conductive material can include silver, copper, gold, or any other conductive material or combination of materials.

In some examples, an adhesive used to secure the hemostatic patch 14 to a patient may be positioned on the skin-facing surface of the outer coating. The outer coating may, in some examples, having a diameter of about 2.5 centimeters (cm) or less.

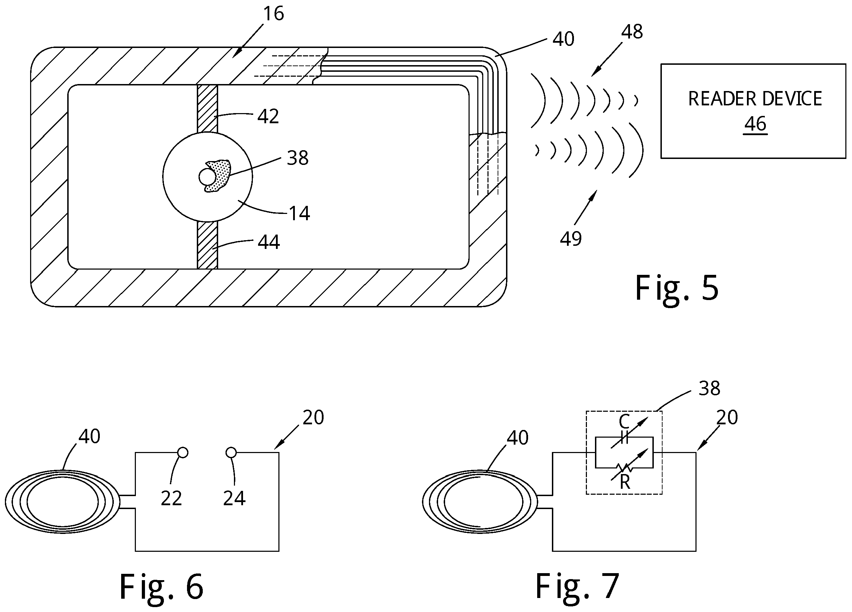

As shown in FIG. 5, in other examples, a radio frequency antenna 40 may be positioned around an outer periphery of the protective cover 16, and may be in electrical communication with the contacts 22, 24 of hemostatic patch via respective electrically conductive traces 42, 44. In some examples, the radio frequency antenna 40 may have a relatively low profile, such that it does not protrude much, if any, from an outer surface of the protective cover 16.

Also shown in FIG. 5 is a reader device 46, which may also be referred to as an interrogator. The reader device 46 may be a handheld device that includes circuitry that generates and transmits an electromagnetic field 48 that, when within relatively close range of the antenna 40, energizes the antenna 40. As discussed above, the antenna 40 may emit a signal 49 when energized at a particular resonant frequency. The reader device 46 may interrogate any electrical circuit 20 of a pathogen detection sensor, whether the electrical circuit 20 is attached to the hemostatic patch 14, the protective cover 16, the access line 12, or another substrate of vascular access system 10. In some examples, the antenna 40 may be an RFID antenna and the reader device 46 may be an RFID reader.

FIG. 6 is a schematic diagram showing the electrical circuit 20, including the electrical contacts 22, 24, and the radio frequency antenna 40, where the electrical circuit 20 is in a first impedance state. The example first impedance state shown in FIG. 6 is an open circuit state. In the open circuit state, the radio frequency antenna 40 does not provide a response signal to an interrogation signal transmitted by the reader device 46.

FIG. 7 is a schematic diagram showing the electrical circuit 20, including the electrical contacts 22, 24, and the radio frequency antenna 40, where the electrical circuit 20 is in a second impedance state. The example second impedance state shown in FIG. 7 is a closed circuit state. In particular, the circuit 20 is closed by the bacterial biofilm 38, which is positioned to the electrically connect contacts 22, 24. In the closed circuit state, the antenna 40 provides a response signal to an interrogation signal transmitted by the reader device 46.

In other examples, instead of, or in addition to, the radio frequency antenna 40, a pathogen detection sensor can communicate the presence of pathogens at or near the vascular access site 18 using an audible and/or visual notification. For example, the communication module 26 (FIG. 2) may include a small battery and a LED, which may be placed in a substrate of the vascular access system 10 that is externally facing, e.g., the protective cover 16 or a cap for access line 12, and in electrical communication with the contacts 20, 22. When the bacterial biofilm electrically connects the electrical contacts on the first surface 30 and the second surface 32, thereby completing the circuit, the LED may be turned on, thereby alerting a care provider of the presence of pathogens at the vascular access site 18.

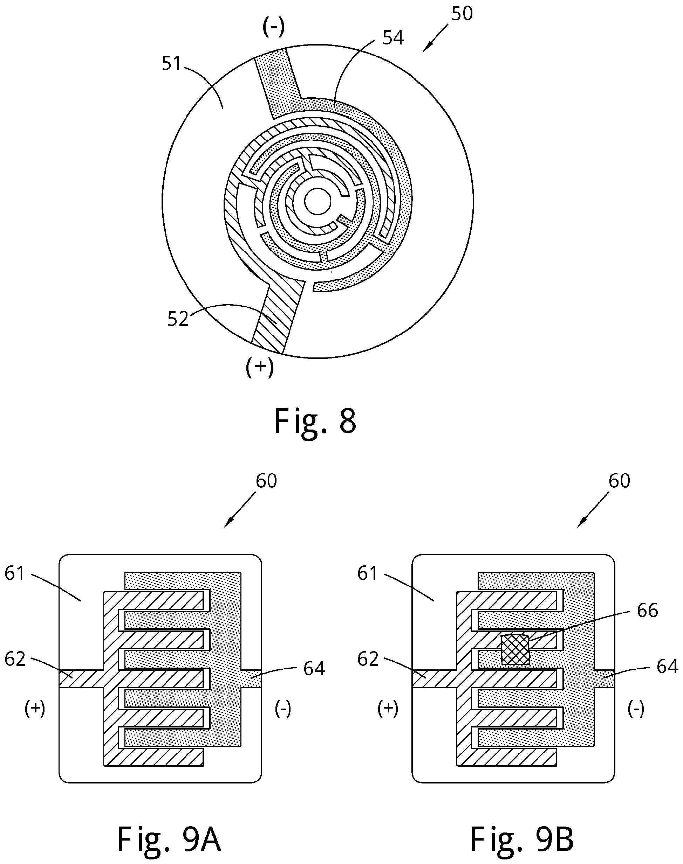

Although the electrical contacts 22, 24 in the form of a continuous coating of an electrically conductive material on the surfaces 30, 32 of the hemostatic patch 14 are described with respect to FIGS. 3 and 4, the electrical contacts 22, 24 may each have another configuration in other examples. FIG. 8 illustrates a side elevation view of an example hemostatic patch 50, which is like the hemostatic patch 14, but rather than including a continuous electrically conductive coating on top and bottom surfaces, the hemostatic patch 50 includes electrically conductive traces 52, 54 on a skin facing surface 51 of the hemostatic patch 50. The skin facing surface 51 may be, for example, a surface of the patch 50 intended to fact towards a patient's skin when the patch 50 is applied onto the patient's skin.

The electrically conductive trace 52 may be an example of the electrical contact 22 of the electrical circuit 20 (FIG. 2) and the electrically conductive trace 54 may be an example of the electrical contact 24 of the electrical circuit 20. The electrically conductive traces 52, 54 are each defined by electrically conductive material deposited on or otherwise formed on an electrically insulative surface of the hemostatic patch 50. The electrically conductive material can be any suitable electrically conductive material, such as a biocompatible metal. In some examples, the electrically conductive traces 52, 54 are each defined by silver, which, as discussed above, is a natural antimicrobial material.

The electrically conductive trace 52 defines a first pattern that extends from one side of the outer perimeter of the hemostatic patch 50 towards a geometric center of the surface 51 of the hemostatic patch 50, and the electrically conductive trace 54 defines a second pattern that extends from a different side of the outer perimeter of the hemostatic patch 50 towards the geometric center of the surface 51. The patterns of the traces 52, 54 are arranged relative to each other such that the electrically conductive traces 52, 54 do not directly contact each other and are physically separated from each other by electrically nonconductive material. As a result, the electrically conductive traces 52, 54 are electrically isolated from each other in the absence of a separate electrical connection electrically connecting the traces 52, 54.

By creating a space between the traces 52, 54, the electrical circuit 20 may be in a first impedance state, i.e., an open state in the example shown in FIG. 8, when there are no pathogens present on the surface 51 and the electrically connecting traces 52, 54. As a bacterial biofilm begins to form, it may eventually grow to be large enough to contact both the traces 52, 54. At this time, the electrical circuit 20 changes to a second impedance state, i.e., a closed state in the example shown in FIG. 8. The electrical circuit 20 can generate a notification to alert care providers of the second impedance state, e.g., using any of the techniques described above with respect to the hemostatic patch 14.

The pattern of the electrically conductive traces 52, 54 may be selected to define a plurality of spaces (or "gaps") between the traces 52, 54 that are distributed across the surface 51 of the hemostatic patch 50. By distributing the spaces across the surface 51, the possibility that a bacterial biofilm may electrically connect the traces 52, 54 may be increased relative to an arrangement in which the traces 52, 54 there are fewer spaces between the traces 52, 54. In this way, the arrangement of the electrically conductive traces 52, 54 may be selected to increase the sensitivity of a pathogen detection sensor that includes the traces 52, 54.

In other examples, in addition to, or instead of the hemostatic patch 50, a protective cover of a vascular access system may include the electrically conductive traces 52, 54 in the configuration shown in FIG. 8 or in another configuration. FIGS. 9A and 9B illustrate another example configuration of electrically conductive traces that may define part of the electrical circuit 20. A protective cover 60, which may be an example of the protective cover 16 of the vascular access system 10, includes electrically conductive traces 62, 64 on a skin facing surface 61 of the cover 60. The electrically conductive trace 62 may be an example of the electrical contact 22 of the electrical circuit 20 (FIG. 2) and the electrically conductive trace 64 may be an example of the electrical contact 24 of the electrical circuit 20. The electrically conductive traces 62, 64 do not directly contact each other, such that the electrically conductive traces 62, 64 are electrically isolated from each other in the absence of a separate electrical connection electrically connecting the traces 62, 64.

In some examples, the electrically conductive traces 62, 64 each define fingers that are positioned between fingers of the other electrically conductive trace and separated from fingers of the other trace other by electrically nonconductive material. The interspaced finger arrangement of the electrically conductive traces 62, 64 may increase a surface area over which the electrical circuit 20 may detect the presence of pathogens, e.g., compared to the electrical contact 30, 32 arrangement of the hemostatic patch 14 shown in FIGS. 3 and 4.

In the example shown in FIG. 9A, the electrical circuit 20 is in a first impedance state, in which the electrically conductive traces 62, 64 are not electrically connected to each other. As shown in FIG. 9B, however, when a bacterial growth 66 forms on the skin facing surface 61 of the protective cover 60, the bacterial growth 66 may electrically connect the electrically conductive traces 62, 64. When the electrically conductive traces 62, 64 are electrically connected to each other, the electrical circuit 20 is in a second impedance state. Accordingly, the second impedance state of the electrical circuit 20 may indicate the presence of the bacterial growth 66 proximate the vascular access site 18 when the protective cover 60 is applied to the patient's skin proximate the vascular access site 18, e.g., over the vascular access site 18.

In other examples, in addition to, or instead of the protective cover 60, the hemostatic patch 14 may include the electrically conductive traces 62, 64 shown in FIGS. 9A and 9B.

In some examples described herein, e.g., in FIGS. 8-9B, the electrical circuit 20 is on a skin-facing surface of a vascular access system substrate or on a surface that may be exposed to electrically conductive fluids (e.g., blood or perspiration). In order to reduce the possibility that an electrically conductive medium (e.g., skin, blood, perspiration, and the like) other than a pathogen growth may change the electrical circuit 20 from the first impedance state to a second impedance state, an electrically insulative material may be positioned between the patient's skin and the electrical contacts 22, 24. The electrically insulative material allows for the passage of a bacterial biofilm or other pathogen growth.

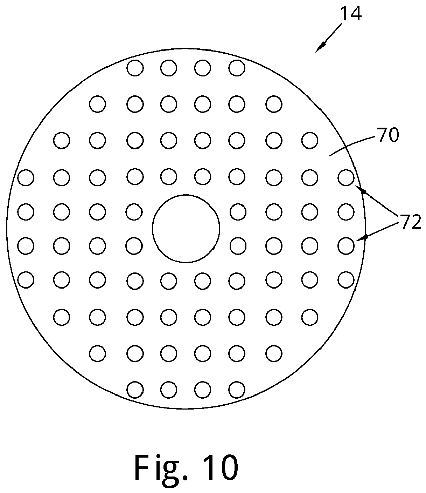

FIG. 10 illustrates the hemostatic patch 14 and an example electrically insulative layer 70 positioned on the same side of the hemostatic patch 14 as the electrical contacts 22, 24 of the electrical circuit 20 (FIG. 2). The electrically insulative layer 70 may be formed from an electrically insulating material (e.g., may consist essentially of an electrically insulative material), such as an electrically insulative plastic (e.g., polyimide) or silicone. The electrically insulative layer 70 is positionable between the electrical contacts 22, 24 and the skin of a patient when the hemostatic patch 14 is applied to the patient's skin.

The electrically insulative layer 70 defines a plurality of openings 72. At least one the openings 72 exposes the electrical contact 22 and at least one other opening 72 exposes the electrical contact 24. A bacterial biofilm may grow on the electrically insulative layer 70 when the layer 70 is positioned adjacent the patient's skin proximate the vascular access site 18. When the bacterial biofilm extends through one or more openings 72 defined by the electrically insulative layer 70, the biofilm may contact the electrical contacts 22, 24, thereby electrically connecting the electrical contacts 22, 24 and changing the electrical circuit 20 from a first impedance state to a second impedance state.

Although the electrically insulative layer 70 is shown in FIG. 10 as being positioned on the hemostatic patch 14, in other examples, another component of the vascular access system 10, such as the protective cover 16, can include the electrically insulative layer 70.

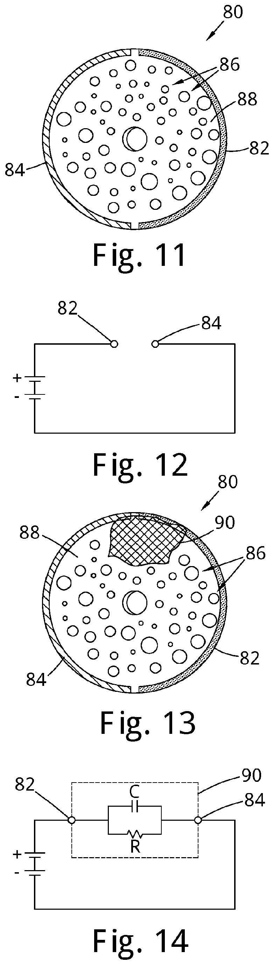

The electrical circuit 20 can include other configurations in other examples. FIG. 11 illustrates a side elevation view of another example hemostatic patch 80, which is like the hemostatic patch 50, but rather than including the electrically conductive traces 52, 54 that are positioned to be relatively close to each other, the hemostatic patch 80 includes a first electrical contact 82, a second electrical contact 84, and electrically conductive particles 86 in an electrically nonconductive substrate 88. The electrically conductive particles 86 are positioned on a surface of the hemostatic patch 80 between the electrical contacts 82, 84.

The electrically conductive particles 86 are dispersed throughout the electrically nonconductive substrate 88 and are placed so as not to be in electrical contact with each other in the absence of an electrically conductive connection (e.g., a bacterial biofilm) external to the patch 80. The electrically conductive particles 86 can be any suitable electrically conductive material, such as silver, gold, or another biocompatible metal, which can be applied to the patch 80 using any suitable technique, such as via a coating or by embedding the particles in the nonconductive substrate 88. The nonconductive substrate 88 may be a substrate that forms the hemostatic patch 80, or may be a separate from the patch 80 and applied to the patch 80.

As discussed above, due to the anti-microbial properties of silver, silver particles may not only be used to detect the presence of pathogens, but may also act as an anti-microbial agent. In some examples, the hemostatic patch 80 includes a uniform, silver-based anti-microbial coating where it is known that the silver eluting particles of the coating are not in electrical contact with each other prior to interacting with a bacterial biofilm.

As shown in FIG. 13, a bacterial biofilm 90 may grow on the skin facing surface of the hemostatic patch 80. As the bacteria grow, the biofilm 90 may eventually grow to be large enough to extend between the electrical contacts 82, 84. The bacterial biofilm 90 in combination with the electrically conductive particles 86 electrically connect the contacts 82, 84 to change the electrical circuit 20 from a first impedance state (shown conceptually in FIG. 12) to a second impedance state (shown conceptually in FIG. 14). The pathogen detection sensor including the electrical circuit 20 can notify a care provider of the second impedance state of the electrical circuit 20 using any of the techniques described above.

In some examples, to minimize false positive detections of pathogens attributable to the natural conductivity of a patient's skin surface, the hemostatic patch 80 may include an electrically insulative layer, such as the layer 70 described above with respect to FIG. 10.

In other examples, in addition to, or instead of the hemostatic patch 14, the protective cover 16 or another substrate of the vascular access system 10 can include a sensor that detects the presence of pathogens at or near the vascular access site 18. For example, the protective cover 16 or another substrate of the vascular access system 10 may include any of the configurations of the electrical circuit 20 described in FIGS. 3-7 and 11-14 with respect to hemostatic patches.

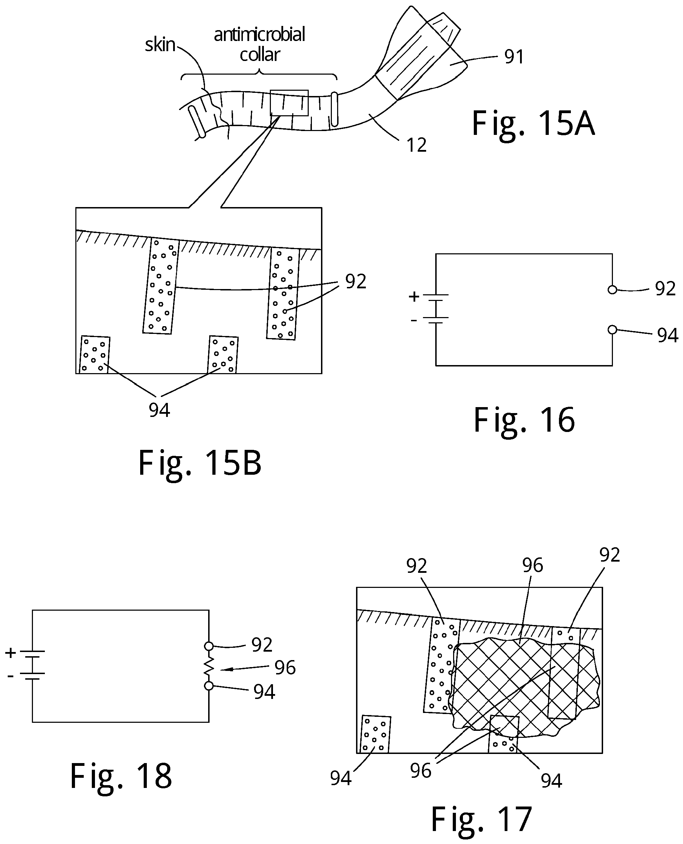

In some examples, in addition to or instead of including at least part of the electrical circuit 20 of a pathogen detection sensor in the hemostatic patch 14 or the protective cover 16 to monitor the insertion site for pathogens, an outer surface of the access line 12 (or another indwelling device) may include at least the electrical contacts 22, 24 of the electrical circuit 20 (FIG. 2). For example, as shown in FIGS. 15A and 15B, an outer surface of the access line 12 can include electrical conductive traces 92, 94, which may be examples of the electrical contacts 22, 24, respectively, of the electrical circuit 20 (FIG. 2). FIG. 15A illustrates a side elevation view of the access line 12 and a luer fitting 91, and FIG. 15B shows a zoomed-in side elevation view of a portion of the access line 12 shown in FIG. 15A. The luer fitting 91 may be used to fluidically connect the access line 12 to another fluid conduit, such as a medicine line, in order to deliver fluids through the access line 12 and into vasculature of a patient when the access line 12 is introduced through skin (shown in FIG. 15A) of a patient and into vasculature of the patient.

The electrically conductive trace 92 may be an example of the electrical contact 22 of the electrical circuit 20 (FIG. 2) and the electrically conductive trace 94 may be an example of the electrical contact 24 of the electrical circuit 20. The electrical conductive traces 92, 94 may be defined by any suitable biocompatible electrically conductive material, such as silver or another biocompatible metal. The traces 92, 94 may define a flexible circuit that is positioned a portion of outer surface of the access line 12 that extends through the skin of a patient. In some examples, the flexible circuit extends over a 2 cm to 4 cm span of access line 12. The flexible circuit could be in addition to or in place of already existing anti-microbial coatings on the access line 12. For example, the electrically conductive traces 92, 94 may be applied to an antimicrobial collar, as shown in FIG. 15A.

The electrical conductive traces 92, 94 are not in direct contact with each other, such that the electrically conductive traces 92, 94 are not electrically connected to each other in the absence of a separate electrical connection electrically connecting the traces 92, 94. For example, the electrically conductive traces 92, 94 may each define fingers that are interleaved with fingers of the other trace, as shown in FIG. 15B. The access line 12 may include other configurations of the traces 92, 94 in other configuration.

FIG. 16 is a circuit diagram conceptually illustrating a first impedance state of the electrical circuit 20 when the electrically conductive traces 92, 94 are not electrically connected. The first impedance state is conceptually shown in FIG. 16 as an open circuit state. However, due to the nature of use of the access line 12, when the access line 12 is introduced into vasculature of a patient, blood and other electrically conductive substances may come into contact with the electrically conductive traces 92, 94 and may electrically connect the traces 92, 94. Thus, in some cases, the electrical circuit 20 may be in a closed state, despite the absence of a bacterial biofilm electrically connecting the traces 92, 94. The first impedance state may, therefore, not be an open circuit state, but, rather, may be some closed circuit state having a first impedance value. The first impedance value may also be referred to as a baseline impedance value that is indicative of a state in which a detectable bacterial biofilm is not present on the access line 12. In some examples, a processor of the pathogen detection sensor or another processor may determine the baseline impedance value when the access line 12 is first introduced into the patient. However, in other examples, the baseline impedance value may be determined at other times.

As shown in FIG. 17, a bacterial biofilm 96 may build-up on the access line 12 and may define an electrical connection between the traces 92, 94, thereby closing the electrical circuit 20 and changing electrical circuit to a second impedance state. The second impedance state is conceptually shown in FIG. 18. In the second impedance state, the electrical circuit 20 has a lower impedance than the baseline impedance.

Any suitable system and technique may be used to provide a notification of the second impedance state of the electrical circuit 20 when the electrical circuit 20 is on the access line 12 or another substrate of the vascular access system 10 that may extend through the patient's skin and into vasculature. For example, any of the notification systems and techniques described above may be used. In addition to, or instead of, the systems and techniques described above, in some examples, the electrically conductive traces 92, 94 may be electrically connectable to electrical contacts on a cap that can, in some examples, snap, screw, or otherwise connect onto a proximal end of the access line 12 that remains external to the patient when a distal end of the access line 12 is introduced into vasculature of the patient. The cap may include, for example, at least part of communications module 26 (FIG. 2) of the electrical circuit 20, such as a radio frequency antenna and/or electronics (e.g., a power source and light emitter) for providing a visual cue to alert a care provider to the detection of the second impedance state of the circuit 20, and, therefore, pathogen-related issues. The cap may allow a care provider to check for the presence of pathogens before the access line 12 is used to deliver a therapeutic agent to the patient.

In some examples, the cap can close off a lumen of the access line 12 when the access line 12 is not in use. In addition, or instead, the cap can be part of a medicine line that fluidly connects to the access line 12 to deliver a therapeutic agent to the patient.

In some examples, a method includes a method of making or using any part of the vascular access systems or pathogen detection sensors described herein.

Various examples have been described. These and other examples are within the scope of the following claims.

The invention may be described by reference to the following numbered paragraphs:-
1. A pathogen detection system comprising:
   an electrical circuit changeable, from a first impedance state to a second impedance state, in response to a pathogen, the electrical circuit comprising:
   a first electrical contact; and
   a second electrical contact electrically connectable to the first electrical contact by the presence of a pathogen extending between the first electrical contact and the second electrical contact, wherein the electrical circuit is in the second impedance state when the first and second electrical contacts are electrically connected to each other through the presence of the pathogen.
2. The pathogen detection system of paragraph 1, wherein the electrical circuit comprises a flexible circuit.
3. The pathogen detection system of paragraph 1, wherein the first electrical contact and the second electrical contact each comprise respective electrically conductive traces.
4. The pathogen detection system of any of paragraph 1, wherein the first electrical contact and the second electrical contact each comprise a biocompatible metal.
5. The pathogen detection system of paragraph 4, wherein the biocompatible metal comprises at least one of silver and gold.
6. The pathogen detection system of any of paragraph 1, wherein the electrical circuit further comprises electrically conductive particles within an electrically nonconductive substrate, the electrically conductive particles disposed between the first electrical contact and the second electrical contact.
7. The pathogen detection system of paragraph 6, wherein the conductive particles comprise a biocompatible metal.
8. The pathogen detection system of paragraph 7, wherein the biocompatible metal comprises at least one of silver and gold.
9. The pathogen detection system of any of paragraph 6, further comprising a substrate having a first side and a second side opposite the first side, the substrate disposed between the first electrical contact and the second electrical contact.
10. The pathogen detection system of any of paragraph 6, wherein the substrate defines an opening extending from the first side of the substrate to the second side of the substrate, the first electrical contact comprising a first layer of conductive material on the first side of the substrate, and the second electrical contact comprising a second layer of conductive material on the second side of the substrate, wherein the first and second electrical contacts are electrically connectable to one another through growth of the pathogen through the opening.
11. The pathogen detection system of any of paragraph 1, wherein the first and second electrical contacts are on a same one of the first side or the second side of the substrate.
12. The pathogen detection system of any of paragraph 6, wherein substrate comprises a protective cover.
13. The pathogen detection system of any of paragraph 6, wherein the substrate comprises a hemostatic patch.
14. The pathogen detection system of any of paragraph 6, further comprising an access line coupled to the substrate, wherein the access line is introducible into vascular of the patient.
15. The pathogen detection system of paragraph 14, wherein the substrate comprises an antimicrobial collar disposed about a circumference of the access line, the first electrical contact and the second electrical contact each positioned along the antimicrobial collar.
16. The pathogen detection system of any of paragraph 1, further comprising an electrically insulative layer over the first electrical contact and over the second electrical contact, the electrically insulative layer permeable to growth of the pathogen.
17. The pathogen detection system of any of paragraph 1, wherein the electrical circuit further comprises a radio frequency antenna in electrical communication with the first electrical contact and the second electrical contact.
18. The pathogen detection system of paragraph 17, further comprising a reader device configured to transmit an interrogation signal that energizes the radio frequency antenna, wherein, in response to the interrogation signal, the radio frequency antenna is configured to transmit a status signal to the reader device, and the reader device is configured to determine, based on the received status signal, whether the electrical circuit is in the first impedance state or in the second impedance state.
19. The pathogen detection system of paragraph 17, wherein the radiofrequency antenna is a passive radiofrequency antenna.
20. The pathogen detection system of any of paragraph 17, further comprising a light emitter and a power source, wherein electrical communication between the light emitter and the power source is dependent upon whether the electrical circuit is in the first impedance state or in a second impedance state.

## Claims

1. A pathogen detection system comprising:
an electrical circuit changeable, from a first impedance state to a second impedance state, in response to a pathogen, the electrical circuit comprising:
a first electrical contact; and
a second electrical contact electrically connectable to the first electrical contact by the presence of a pathogen extending between the first electrical contact and the second electrical contact, wherein the electrical circuit is in the second impedance state when the first and second electrical contacts are electrically connected to each other through the presence of the pathogen.

2. The pathogen detection system of claim 1, wherein the electrical circuit comprises a flexible circuit; and/or wherein the first electrical contact and the second electrical contact each comprise respective electrically conductive traces.

3. The pathogen detection system of any of claim 1 or claim 2, wherein the first electrical contact and the second electrical contact each comprise a biocompatible metal.

4. The pathogen detection system of claim 3, wherein the biocompatible metal comprises at least one of silver and gold.

5. The pathogen detection system of any preceding claim, wherein the electrical circuit further comprises electrically conductive particles within an electrically nonconductive substrate, the electrically conductive particles disposed between the first electrical contact and the second electrical contact.

6. The pathogen detection system of any preceding claim, wherein the conductive particles comprise a biocompatible metal; preferably wherein the biocompatible metal comprises at least one of silver and gold.

7. The pathogen detection system of claim 6, further comprising a substrate having a first side and a second side opposite the first side, the substrate disposed between the first electrical contact and the second electrical contact; preferably wherein the substrate defines an opening extending from the first side of the substrate to the second side of the substrate, the first electrical contact comprising a first layer of conductive material on the first side of the substrate, and the second electrical contact comprising a second layer of conductive material on the second side of the substrate, wherein the first and second electrical contacts are electrically connectable to one another through growth of the pathogen through the opening.

8. The pathogen detection system of claim 7, wherein the first and second electrical contacts are on a same one of the first side or the second side of the substrate.

9. The pathogen detection system of claim 7 or claim 8, wherein substrate comprises a protective cover; and/or wherein the substrate comprises a hemostatic patch.

10. The pathogen detection system of any of claims 6 to 9, further comprising an access line coupled to the substrate, wherein the access line is introducible into vascular of the patient; preferably wherein the substrate comprises an antimicrobial collar disposed about a circumference of the access line, the first electrical contact and the second electrical contact each positioned along the antimicrobial collar.

11. The pathogen detection system of any preceding claim, further comprising an electrically insulative layer over the first electrical contact and over the second electrical contact, the electrically insulative layer permeable to growth of the pathogen.

12. The pathogen detection system of any preceding claim wherein the electrical circuit further comprises a radio frequency antenna in electrical communication with the first electrical contact and the second electrical contact.

13. The pathogen detection system of claim 12, further comprising a reader device configured to transmit an interrogation signal that energizes the radio frequency antenna, wherein, in response to the interrogation signal, the radio frequency antenna is configured to transmit a status signal to the reader device, and the reader device is configured to determine, based on the received status signal, whether the electrical circuit is in the first impedance state or in the second impedance state.

14. The pathogen detection system of claim 13, wherein the radiofrequency antenna is a passive radiofrequency antenna.

15. The pathogen detection system of claim 14, further comprising a light emitter and a power source, wherein electrical communication between the light emitter and the power source is dependent upon whether the electrical circuit is in the first impedance state or in a second impedance state.
